# EUROPEAN PATENT SPECIFICATION

(11) **EP 4 247 332 B1**
(45) Date of publication and mention of the grant of the patent: **22.04.2026**
(21) Application number: 21811355.3
(22) Date of filing: 18.11.2021
(51) Int. Cl.: A61K 9/00, A61K 9/06, A61K 47/10, A61K 47/30, A61K 47/34, A61K 45/06, A61K 31/165, A61P 27/16

(54) **NON-AQUEOUS GEL COMPOSITION**
NICHTWÄSSRIGE GELZUSAMMENSETZUNG
COMPOSITION DE GEL NON AQUEUSES

(30) Priority: 19.11.2020 EP 20208665
(43) Date of publication of application: 27.09.2023
(73) Proprietor: Acousia Therapeutics GmbH, 72070 Tübingen (DE)
(72) Inventor: BÖGERSHAUSEN, Ansgar, 79102 Freiburg (DE); LIEBICH, Lena, 79539 Lörrach (DE); RISCHER, Matthias, 60388 Frankfurt (DE)
(74) Representative: Patentanwälte Ruff, Wilhelm, Beier, Dauster & Partner mbB
(86) International application number: PCT/EP2021/082108
(87) International publication number: WO 2022/106523

(56) References cited:
- EP-A1- 3 256 463
- EP-B1- 3 256 463
- WO-A1-2009/023272
- WO-A1-2012/156820
- WO-A1-2019/126783
- WO-A1-2019/140012
- WO-A1-2019/202504
- WO-A1-2019/210107
- WO-A1-2020/112390
- WO-A2-2010/011466
- WO-A2-2018/140792
- WO-A2-2018/158256
- JP-A- 2003 063 927
- US-A1- 2018 000 950
- US-A1- 2018 296 472
- MEHTA DHRUTI P ET AL: "A Review on Pharmaceutical Gel", INTERNATIONAL JOURNAL OF PHARMACEUTICAL SCIENCES, vol. 1, no. 1, 1 September 2015 (2015-09-01), pages 33 - 47, XP055793084, Retrieved from the Internet <URL:https://www.researchgate.net/profile/Hemendrasinh-Rathod/publication/286451492_A_Review_on_Pharmaceutical_Gel/links/566a473308ae430ab4f774b5/A-Review-on-Pharmaceutical-Gel.pdf>
- TRIPATHI: "Pharmaceutical gels: In summarized form", PHARMATUTOR PHARMACY INFOPEDIA, 1 July 2013 (2013-07-01), pages 1 - 20, XP055793072, Retrieved from the Internet <URL:https://www.pharmatutor.org/articles/pharmaceutical-gels-in-summarized-form> [retrieved on 20210407]
- S. S. SAGIRI ET AL: "Organogels as Matrices for Controlled Drug Delivery: A Review on the Current State", SOFT MATERIALS, vol. 12, no. 1, 2 August 2013 (2013-08-02), US, pages 47 - 72, XP055746608, ISSN: 1539-445X, DOI: 10.1080/1539445X.2012.756016
- LEI MENGTING ET AL: "Non-aqueous foams formed by whipping diacylglycerol stabilized oleogel", FOOD CHEMISTRY, ELSEVIER LTD, NL, vol. 312, 17 December 2019 (2019-12-17), XP085977920, ISSN: 0308-8146, [retrieved on 20191217], DOI: 10.1016/J.FOODCHEM.2019.126047
- AYESHA SYEDA AHMED UN NABI ET AL: "Pharmaceutical Gels: A Review", RADS JOURNAL OF PHARMACY AND PHARMACEUTICAL SCIENCES, vol. 4, no. 1, 1 June 2016 (2016-06-01), pages 40 - 48, XP055793086
- ANDERSON KATIE: "Dr. Straetmans Introduces Natural Oil Thickener and Cold-processable Emulsifier", COSMETICS & TOILETRIES, 2 April 2013 (2013-04-02), pages 1 - 2, XP055793276, Retrieved from the Internet <URL:https://www.cosmeticsandtoiletries.com/formulating/function/viscositymod/Natural-Oil-Thickener-and-Cold-processable-Emulsifier-201086511.html> [retrieved on 20210407]
- ANONYMOUS: "ABWAX� POLYETHYLENE WAX Supplied by ROELMI HPC", SPECIALCHEM COSMETICS INGREDIENTS, 18 February 2021 (2021-02-18), pages 1 - 2, XP055793463, Retrieved from the Internet <URL:https://cosmetics.specialchem.com/product/i-roelmi-hpc-abwax-polyethylene-wax> [retrieved on 20210408]
- VINTILOIU ET AL: "Organogels and their use in drug delivery - A review", JOURNAL OF CONTROLLED RELEASE, ELSEVIER, NL, vol. 125, no. 3, 7 November 2007 (2007-11-07), pages 179 - 192, XP022432121, ISSN: 0168-3659, DOI: 10.1016/J.JCONREL.2007.09.014

## Description

### TECHNICAL FIELD AND PRIOR ART

The present invention relates to a novel non-aqueous gel composition, in particular useful as a carrier of pharmaceutically active agents. The present invention also relates to a discharging device comprising this non-aqueous gel composition and to the use of this non-aqueous gel composition as a component of a pharmaceutical composition or of a medicament.

Because of their viscosity properties gel compositions are often used as carriers for topical and oral administration of pharmaceutically active agents. Important for the administration of gel compositions are e.g. specific viscosity properties that on the one hand allow sufficient syringeability, i.e. the capability of being dispensed from, and/or drawn into, a syringe, and on the other hand a good adhesion of the composition at organic tissues at about body temperature, in particular for a period of at least 3 days, preferably of at least 5 days, after a single administration at organic tissue.

Therefore, thermoreversible aqueous gel compositions, whose viscosity changes at a characteristic temperature, comprising mainly copolymers in an amount sufficient to provide a gelation temperature at about body temperature, are often used.

However, despite the good viscosity properties aqueous gel compositions show the disadvantage of not being able to carry pharmaceutically active agents that have relatively poor water solubility properties, if those pharmaceutically active agents are not to be administered as a suspension in the gel.

Non-aqueous gel compositions offer a good alternative as carriers, for said pharmaceutically active agents that are having relatively poor water solubility properties. Further, because of their lipophilic character non-aqueous gel compositions can create the possibility that the pharmaceutically active agent can at least partly pass through cell membranes.

However, when it comes to viscosity properties that would allow a good syringeability and a good adhesion at organic tissues conventional non-aqueous gel compositions lack those thermoreversible properties.

Especially for the administration of gel compositions at internal organs, there is a need to administer the pharmaceutical composition with a syringe. Further, internal organs such as eyes, ears, nose, mouth, lip, vagina, urethral opening and anus are mostly covered by mucus or mucous membranes which makes adhesion (mucoadhesion) challenging.

Therefore, conventional non-aqueous gel compositions cannot facilitate both a proper viscosity, which would allow a good syringeability, and a good adhesion at organic tissues.

Thermoreversible aqueous gel compositions are known from EP 3501521 A1 and WO 2019210107. The corresponding compositions comprise pharmaceutically active agents like cisplatin, carboplatin, oxiplatin or corticosteroids, in particular Dexamethason or JNK (c-Jun N-terminal kinase) inhibitors, for the treatment or prevention of drug induced ototoxicity.

US 20180000950 A1 describes a non-aqueous gel composition comprising a therapeutic agent, triglycerides and at least one viscosity modulating agent. The triglycerides are present in an amount that is sufficient to stabilize the therapeutic agent for injection into the ear. Preferably the at least one viscosity modulating agent is silicon dioxide.

There are some patent documents comprising non-aqueous thermoreversible gels for the use in the treatment of different diseases, for example WO 2014076569 A2 and EP 0530965 A1.

EP 0530965 A1 describes a composition for nasal application comprising at least one sexual hormonic drug, at least one lipophilic carrier and a surfactant. Further, the composition comprises a viscosity regulating agent which is colloidal silicon dioxide.

US 2013150410 A1 describes a method of treating otic disorders selected from a group consisting of Meniere's Disease, endolymphatic hydrops, progressive hearing loss, dizziness, Vertigo and tinnitus, comprising transtympanic administration of a sterile pharmaceutical composition into the ear. The composition comprises a multiparticulate ion channel modulating agent such that sustained release of the ion channel modulating agent into the ear occurs for a period of at least 5 days.

US 2019038469 A1 claims a system for delivering a therapeutic composition to the inner ear. Further a system comprising a delivery sheath having a lumen and a cannula configured to be inserted through the lumen of the delivery sheath is described. The cannula has a lumen for delivery of the therapeutic composition and a distal tip for piercing the tympanic membrane for delivery of the therapeutic composition to the round window membrane so as to have the composition adhere to the round window membrane. Further, the composition comprises a therapeutic agent for treating an inner ear disorder and a thixotropic material which allows the therapeutic composition to exist in a liquid form while being injected through the cannula.

WO 2018158256 discloses pharmaceutical compositions which may be in the form of a gel that can be applied into the middle ear and release the active agent over a longer period due to their jelly-like consistency.

It is an object of the present invention to provide a novel gel composition, which has superior properties, compared to conventional gel compositions.

### SUMMARY OF THE INVENTION

The above object and further objects are solved by a non-aqueous gel composition for transtympanic administration according to claim 1, a syringe according to claim 8 and a non-aqueous gel composition for use as a pharmaceutical composition or medicament according to claim 9. Preferred embodiments are defined in the dependent claims. The said non-aqueous gel composition is especially suitable for a transtympanic administration and comprises a pharmaceutically active agent having the formula I defined in claim 1, which has a relatively poor water solubility.

The present invention provides a non-aqueous gel composition comprising
- castor oil, present in an amount from 85 to 95% by weight, based on the total weight of the composition,
- Labrafil^{®}, which is an oleoyl macrogolglyceride and is present in an amount from 2 to 6 % by weight, based on the total weight of the composition,
- at least one thickener, preferably silicon dioxide, more preferably colloidal silicon dioxide, present in an amount from 1 to 6 % by weight, based on the total weight of the composition, and wherein
- the viscosity of the composition at a temperature of 25°C is between 1400 and 2400 mPas, preferably between 1400 and 1800 mPas,
- the non-aqueous gel composition further comprises at least one pharmaceutically active agent having the formula I defined ni claim 1, and having a mean particle size of 0,01 to 100 µm.

In this composition castor oil has the function of a lipophilic carrier and the oleoyl macrogolglyceride, preferably Labrafil^{®}, has the function of a surfactant.

In a preferred embodiment of the invention the inventive non-aqueous gel composition is sterile.

In another preferred embodiment of the invention the inventive non-aqueous gel composition is an oleogel.

The terms used in the claims and in the overall description are defined as follows.

The term "non-aqueous composition" as used herein, means a composition which is free of water or substantially free of water.

The term "oleogel" as used herein, means a semi-solid non-aqueous gel composition having viscoelastic properties, based on lipids, in particular fats.

The term "lipophilic carrier" as used herein, means a substrate, preferably a lipophilic substrate, used in the process of pharmaceutically active compound delivery, which serves to improve the administration of pharmaceutically active agents of varying lipophilicity.

The term "surfactant" as used herein, means a compound that lowers the surface tension between two liquids or between a liquid and a solid in a composition, enabling or supporting the formation of a dispersion and improving wettability of the composition. The surfactant can also act as detergent, emulsifier, and foaming agent.

The term "thickener" as used herein, means a substance which can increase the viscosity of a liquid composition without substantially changing its other properties. Preferably the thickener acts as gallant forming a cohesive internal structure, especially a gel and is therefore also known as gelling agent.

The term "region or structure of the ear" as used herein, means a region of the ear, in particular the middle ear region of the ear, or a structure of the ear, in particular the round window structure of the ear, wherein said structure of the ear is a part of said region of the ear.

The term "transtympanic administration" as used herein, means an injection through the tympanic membrane into the middle ear for administration in the middle ear, in particular on the round window membrane. Wherein said administration on the round window membrane is to facilitate diffusion across the round window membrane.

The term "sustainable release gel" as used herein, means a gel composition comprising at least one pharmaceutically active compound, which provides an equally, sustained and controlled liberation, in particular release, of the at least one pharmaceutically active compound into a body, in particular a human body, and its tissues.

The term "multiparticulate" as used herein, means a plurality of particles of at least one pharmaceutically active agent, wherein the particles are of the same size or of different size.

The term "micronized" as used herein, means a substance, in particular a pharmaceutically active agent, which is reduced in size to particles which are measured in µm.

The term "sterile" as used herein, means free from living germs or microorganisms, in particular aseptic.

The term "mucoadhesion" as used herein, means an adhesion at organic tissues which are mostly covered by mucus or mucous membranes such as eyes, ears, nose, mouth, lip, vagina, urethral opening and anus.

The term "thermoreversible gel" as used herein, means a gel composition, whose viscosity changes at a characteristic temperature. This thermal behavior includes, that the property of the gel is not affected by repeated heating and cooling and that the viscosity of the gel is reversible to its initial state.

In a preferred embodiment of the invention the viscosity of the inventive non-aqueous gel composition at a temperature of 25°C is between 1400 and 2300 mPas, more preferably between 1600 and 2000 mPas. Within the last-mentioned range viscosities between 1700 and 1800 mPas are even further preferred.

According to the invention the viscosity of the inventive non-aqueous gel composition at a temperature of 37°C is preferably between 500 and 1200 mPas, more preferably between 700 and 1000 mPas.

In contrast to conventional gel compositions, the inventive non-aqueous gel composition has the advantage to provide a thermoreversible gel composition, whose viscosity changes at a characteristic temperature.

The thermoreversible viscosity properties mentioned above provide a non-aqueous gel composition with a viscosity making it especially useful in a pharmaceutical composition or in a medicament for topical administration. A viscosity is provided that is sufficient for a syringeability at (pharmaceutical) room temperature between 15°C and 25°C, i.e. the capability of being dispensed from, and/or drawn into, a syringe, and a viscosity that allows good adhesion of the composition for topical administration at organic tissue at about body temperature normally between 35°C and 38°C, in particular for a period of at least 3 days, preferably of at least 5 days, e.g. after a single administration at organic tissue.

This is especially advantageous for the administration of gel compositions at internal organs, where both a need to administer the composition with a syringe, and a need for a good adhesion, especially a good mucoadhesion is given.

The inventive non-aqueous gel composition preferably comprises a viscosity and gel structure useful to be applied via a suitable needle diameter of 18 to 27 gauge (Birmingham gauge), more preferable of 20 to 23 gauge, in particular of 20 gauge. The inventive non-aqueous gel composition will be also suitable of being applied in small volumes from 20 µL to 200 µL, e.g. to the inner ear and/or the middle ear.

According to the invention viscosity normally is measured with a viscometer, in particular a rheometer. A rheometer usually is a laboratory device measuring flow characteristics of a liquid, suspension or slurry in response to applied forces. A common device type is a so called shear rheometer, in which a shear stress is applied to the corresponding material. Devices which can be used to measure the viscosity of the non-aqueous gel compositions according to the invention are rheometers from Brookfield (AMETEK Brookfield).

In another embodiment of the invention the inventive non-aqueous gel composition is preferably free of polymers, including copolymers.

In another embodiment of the invention the castor oil of the inventive non-aqueous gel composition can comprise at least one further lipophilic carrier preferably at least one glyceride ester selected from a group comprising monoglyceride ester, diglyceride ester, triglyceride ester and mixtures thereof, wherein preferably the glyceride ester is a triglyceride ester.

According to the invention the at least one glyceride ester preferably comprises at least one unsaturated or saturated, preferably unsaturated, fatty acid with a chain length of the fatty acid between 16 to 20 C-atoms, preferably of 18 C-atoms.

In a preferred embodiment of the invention the at least one fatty acid is preferably selected from a group comprising ricinoleic acid, oleic acid, stearic acid, linoleic acid, palmic acid.

In another preferred embodiment of the invention the at least one further lipophilic carrier preferably comprises a triglyceride ester comprising three unsaturated fatty acids with a chain length of the fatty acid containing 18 C-atoms, wherein the fatty acid is ricinoleic acid. According to the invention the at least one further lipophilic carrier comprising at least one glyceride ester is preferably selected from a group comprising almond oil, linseed oil, canola oil, corn oil, cotton seed oil, palm oil, peanut oil, poppy seed oil, soya bean oil and mixtures thereof. In a preferred embodiment of the invention the at least one further lipophilic carrier comprising at least one glyceride ester is selected from a group comprising natural oil, synthetic oil, semisynthetic oil and mixtures thereof.

In another embodiment of the invention the oleoyl macrogolglyceride, Labrafil^{®}, of the inventive non-aqueous gel composition can comprise at least one further surfactant preferably selected from a group comprising oleoyl macrogolglycerides, acylglycerols, lecithins, polyoxyethylene, polyoxyethylenesorbitans, polyglycerol, polyethyleneglycole and mixtures thereof.

The surfactants described above, especially the oleoyl macrogolglyceride, are especially useful for improving the wettability of the composition to ensure a homogeneous distribution of hydrophobic particles, preferably hydrophobic pharmaceutically active agent particles, in the inventive non-aqueous gel composition.

According to the invention the oleoyl macrogolglyceride is Labrafil^{®}, more preferably Labrafil^{®} M1944 CS. Labrafil^{®} M1944 CS is an oleoyl polyoxy-6-glyceride.

Labrafil^{®} is a registered trademark of Gattefossé. The Labrafil^{®} series includes Labrafil^{®} M 1944 CS, Labrafil^{®} M 2125 CS (linoleoyl Polyoxyl-6 glyceride) and Labrafil^{®} M 2130 CS (lauroyl polyoxyl-6 glyceride).

According to the invention the at least one thickener is preferably silicon dioxide, more preferably colloidal silicon dioxide. In another embodiment of the invention the at least one thickener is preferably selected from a group comprising stearate, colloidal silicon dioxide, mesoporous silicon dioxide and mixtures thereof.

The above mentioned thickeners, especially colloidal silicon dioxide, were found to be especially useful for improving the adhesion properties of the inventive non-aqueous gel composition at organic tissues at about body temperature (normally 35-38°C), in particular for a period of at least 3 days, preferably of at least 5 days, in particular after a single administration at organic tissue. In a preferred embodiment of the invention the at least one thickener is preferably colloidal silicon dioxide.

In another preferred embodiment of the invention the at least one thickener is preferably not dissolved, but dispersed in the inventive non-aqueous gel composition. Thereby a thixotropic gel structure is obtained resulting in a non-aqueous gel composition that shows an increased viscosity for a period of time when a certain shear stress is applied to the composition.

According to the invention castor oil is present in the inventive non-aqueous gel composition in an amount from 85 to 95 % by weight, preferably in an amount of 91 to 94 % by weight, more preferably in an amount of 94 % by weight, based on the total weight of the composition.

In a preferred embodiment the at least one further lipophilic carrier is present in the castor oil in an amount less than 5%.

According to the invention the oleoyl macroglyceride, Labrafil^{®}, is present in the inventive non-aqueous gel composition in an amount from 2 to 6 % by weight, preferably in an amount of 3,5 % by weight, based on the total weight of the composition.

In a preferred embodiment the at least one further surfactant is present in the oleoyl macroglyceride, preferably Labrafil^{®}, in an amount less than 5 %.

The amount of a surfactant, especially an oleoyl macroglyceride, preferably Labrafil^{®}, in the non-aqueous gel composition has been found important to ensure a good wettability on the one hand, without negatively influencing the adhesion properties of the non-aqueous gel composition on the other hand. An amount of at least one surfactant, especially an oleoyl macroglyceride, preferably Labrafil^{®}, as described above, has been found especially useful to assure a good wettability and good adhesion properties of the non-aqueous gel composition.

According to the invention the at least one further thickener, preferably silicon dioxide, more preferably colloidal silicon dioxide is present in the inventive non-aqueous gel composition in an amount from 1 to 6 % by weight, preferably in an amount of 2 % by weight, based on the total weight of the composition.

The amount of the at least one thickener, especially colloidal silicon dioxide, comprised in the inventive non-aqueous gel composition as described above is especially useful to provide sufficient syringeability of the composition at room temperature (see above).

It was found that the inventive non-aqueous gel composition is especially useful for providing an in vivo sustained release of a therapeutically effective amount of pharmaceutically active agents after administration, preferably at internal organs, in particular after transtympanic administration, for a period of at least 3 days, preferably at least 5 days, after a single administration at organic tissue.

According to the invention the inventive non-aqueous gel composition further comprises at least one pharmaceutically active agent having the formula I defined in claim 1.

In an embodiment of the invention, the inventive non-aqueous gel composition is preferably a sustainable release gel, which comprises at least one pharmaceutically active compound, which provides an equally, sustained and controlled liberation, in particular release, of the at least one pharmaceutically active compound into a body, in particular a human body, over a period of at least 3 days, preferably of at least 5 days, more preferably of at least 1 month after a single administration, in particular at organic tissue.

According to the invention the at least one pharmaceutically active agent is preferably present in an amount from 0,05 to 10 % by weight, preferably from 0,1 to 10 % by weight, more preferably from 0,5 to 5 % by weight, more preferably 0,6 % or 3% by weight, alternatively from 3 to 10 % by weight, preferably from 6 to 10 % by weight, based on the total weight of the composition, including the active agent.

The amount of the at least one pharmaceutically active agent as described above, was found to be especially useful for providing a sustainable release gel, releasing the pharmaceutically active agent from the inventive non-aqueous gel composition, for a period of at least 3 days, preferably at least 5 days, after a single administration at organic tissue.

According to the invention the at least one pharmaceutically active agent preferably has a mean particle size of 0,01 to 100 µm, preferably of 5 to 80 µm, more preferably of 20 to 50 µm.

The term "mean particle size" as used herein, means a particle whose dimensions, in particular its diameter, preferably mean diameter, lies in a range of 0,01 to 100 µm, preferably of 5 to 80 µm, more preferably of 20 to 50 µm. In this context, the term "diameter" is to be understood in the case of a spherical particle to mean the diameter of the sphere, i.e. twice the radius of the sphere. In the case of a non-spherical particle, the term "diameter" is to be understood as the largest possible distance that two points along a circumference of the particle can take from each other.

The mean particle size distribution is preferably obtained by laser diffraction. During a laser diffraction measurement a laser beam passes through a dispersed particulate sample and the angular variation in intensity of the scattered light is measured. Large particles scatter light at small angles relative to the laser beam and small particles scatter light at large angles. The angular scattering intensity data is then analyzed to calculate the size of the particles that created the scattering pattern using the Mie theory of light scattering. The particle size is reported as a volume equivalent sphere diameter. The folded optical design in the Mastersizer 3000 (from Malvern Panalytical), which was in particular used for the measurements provides a particle size measurement range from 10 nm up to 3.5 mm using a single optical measurement path. The Mastersizer 3000 uses a sequential combination of measurements with red and blue light sources to measure across the entire particle size range.

The above described mean particle size of the at least one pharmaceutically active agent was found to be especially useful to obtain a homogenous distribution of the at least one pharmaceutically active agent in the non-aqueous gel composition.

In an embodiment of the invention the desired particle size of the at least on pharmaceutically active agent has been preferably obtained by grinding, ball milling, high pressure homogenization, homogenization, micronisation or a combination of at least two of the described methods.

Further, due to the above described particle size of the at least one pharmaceutically active agent the uptake of the at least one pharmaceutically active agent from the inventive composition into a body, after administration at organic tissues, is especially advantageous in comparison to other conventional gel compositions comprising pharmaceutically active agents. Due to the advantageous uptake of the pharmaceutically active agent from the inventive non-aqueous gel composition into the body, lesser amounts of pharmaceutically active agent in the composition are needed to achieve the same therapeutically effect as conventional gel compositions.

The term "uptake of a pharmaceutically active agent into a body" as used herein, means an uptake of a pharmaceutically active agent into a mammal body, in particular a human and/or an animal body, in particular into the perilymph and/or endolymph of a mammal body, in particular of a human and/or of an animal body.

In one embodiment of the invention the inventive non-aqueous gel composition comprises, based on the concentration of the pharmaceutically active agent, a multiparticulate suspension of the pharmaceutically active agent.

In a preferred embodiment of the invention the inventive non-aqueous gel composition preferably comprises a solution of the pharmaceutically active agent.

According to the invention the at least one pharmaceutically active agent preferably has a (low) solubility in water at room temperature (22-26°C) of < 1 mg/mL, preferably < 0,1 mg/mL.

The low solubility of pharmaceutically active agents negatively affects the uptake of the pharmaceutically active agent into the body, in particular in the perilymph and/or endolymph of the body, after administration, in particular at internal organs, e.g. after transtympanic administration. As a consequence, a therapeutic effect cannot be achieved by a single administration, but a local depot formulation has to be developed which uses the intrinsic low solubility of the pharmaceutically active agents in a suitable gel composition to obtain a sustained uptake of the pharmaceutically active agents over the desired time interval.

The inventive non-aqueous gel composition shows the advantage of being especially useful for carrying pharmaceutically active agents, having a (low) solubility as described above, in particular for a topical administration. Further the inventive non-aqueous gel composition can be administered to form a local depot to obtain a sustained uptake of the pharmaceutically active agents for a period of at least 3 days, preferably at least 5 days, in particular after a single administration at organic tissue.

The at least one pharmaceutically active agent is a Biopharmaceutics Classification System Class II (BCS Class II) substance comprising low solubility properties and high permeability properties, wherein the classification is based on the Biopharmaceutics Classification (BCS) System.

The BCS system was introduced for a better characterization of pharmaceutically active agents with low solubility. The BCS system distinguishes four categories based on the solubility and permeability of the pharmaceutically active agent.

According to the invention the at least one pharmaceutically active agent has the formula I: 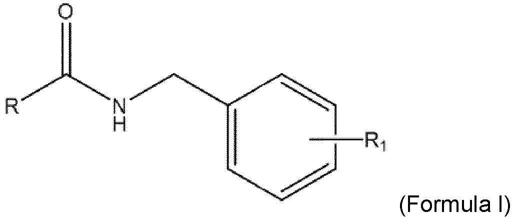 wherein
- R is a unsubstituted cycloalkyl group, in particular bicycloalkyl group, a unsubstituted or substituted phenyl group, or a unsubstituted or substituted thienyl group, wherein said substituted thienyl group or phenyl group is substituted with at least one halogen, preferably selected from a group comprising at least one F- Atom, Cl- Atom, Br- Atom or I-Atom, more preferably at least one F-atom or at least one Cl-atom, and
- R₁ is F, SF₅, CF₃ or OCF₃.

According to the invention the at least one pharmaceutically active agent is preferably selected from a group comprising
(1R,2R,4S)-rel-N-(3-(pentafluorosulfanyl)benzyl)bicyclo[2.2.1]heptane-2-carboxamide, (1S,2S,4R)-N-(3-(pentafluorosulfanyl)benzyl)bicyclo[2.2.1]heptane-2-carboxamide,
or stereoisomers or tautomers of
(1S,2S,4R)-N-(3-(pentafluorosulfanyl)benzyl)bicyclo[2.2.1]heptane-2-carboxamide in particular
the enantiomer (1S,2S,4R)-N-(3-(pentafluoro-λ6-sulfaneyl)benzyl)bicyclo[2.2.1]heptane-2-carboxamide or
the racemic mixture (1SR,2SR,4RS)-N-(3-(pentafluoro-λ6-sulfaneyl)benzyl)bicyclo[2.2.1] heptane-2-carboxamide),
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide, and
p-Fluoro-N-(4-(trifluoromethyl)benzyl)benzamide.

The pharmaceutically active agents listed above belong to a class of compounds which preferably function as potassium channel openers, in particular as openers of the Kv7.4 potassium channel and are therefore especially useful in the treatment of disorders associated with an aberrant potassium activity like Alzheimer's disease and Parkinson's disease. Further disorders are neurologic conditions such as epilepsy or cognitive psychiatric disorders like depression, mania and schizophrenia. In particular, it is known that potassium channels play an important role for the normal function the outer hair cells (OHC) in the organ of the corti. Therefore, the pharmaceutically active agents listed above are promising candidates for the treatment and/or prophylaxis of hearing loss, e.g. before a treatment with an ototoxicity inducing drug.

The pharmaceutically active agent described above, in particular (1SR,2SR,4RS)-N-(3-(pentafluoro-A6-sulfanyl)benzyl)bicyclo[2.2.1]heptane-2-carboxamide (in the following called Compound A), the enantiomer (1S,2S,4R)-N-(3-(pentafluoro-λ6-sulfaneyl)benzyl)bicyclo[2.2.1]heptane-2-carboxamide thereof (in the following called Compound B), show a (low) solubility in water at room temperature (22-26°C) of < 1 mg/mL, preferably < 0,1 mg/mL and can therefore be classified as a BCS class II drug substance.

According to the invention a non-aqueous gel composition is preferred, wherein:
- castor oil is present in an amount from 85 to 95 % by weight, based on the total weight of the composition,
- the oleoyl macrogolglyceride, preferably Labrafil^{®}, is present in an amount from 2 to 6 % by weight, based on the total weight of the composition,
- the at least one thickener, preferably silicon dioxide, more preferably colloidal silicon dioxide, is present in an amount from 1 to 6 % by weight, based on the total weight of the composition,
- the viscosity of the composition at a temperature of 25°C is between 1400 and 2400 mPas, preferably between 1400 and 1800 mPas and
- the at least one pharmaceutically active agent is selected from a group comprising (1R,2R,4S)-rel-N-(3-(pentafluoro-λ6-sulfanyl)benzyl)bicyclo[2.2.1]heptane-2-carboxamide,
   (1S,2S,4R)-N-(3-(pentafluoro-λ6-sulfanyl)benzyl)bicyclo[2.2.1]heptane-2-carboxamide, or stereoisomers or tautomers of
   (1S,2S,4R)-N-(3-(pentafluoro-λ6-sulfanyl)benzyl)bicyclo[2.2.1]heptane-2-carboxamide in particular
   the enantiomer (1S,2S,4R)-N-(3-(pentafluoro-λ6-sulfaneyl)benzyl)bicyclo[2.2.1] heptane-2-carboxamide or
   the racemic mixture (1SR,2S,4R)-N-(3-(pentafluoro-λ6-sulfaneyl)benzyl) bicyclo[2.2.1] heptane-2-carboxamide),
   (1R,2R,4S)-rel-N-(3-(trifluormethyl)benzyl)bicyclo[2.2.1]heptane-2-carboxamide,
   (1R,2R,4S)-rel-N-(3-(trifluoromethoxy)benzyl)bicyclo[2.2.1]heptane-2-carboxamide,
   (1S,2S,4R)-N-(3-(trifluoromethyl)benzyl)bicyclo[2.2.1]heptane-2-carboxamide,
   (1S,2S,4R)-N-(3-(trifluoromethoxy)benzyl)bicyclo[2.2.1]heptane-2-carboxamide,
   p-Chloro-N-(4-trifluoromethoxy)benzyl)benzamide,
   p-Chloro-N-(4-(pentafluorosulfanyl)benzyl)benzamide,
   p-Fluoro-N-(4-trifluoromethoxy)benzyl)benzamide,
   p-Fluoro-N-(4-(pentafluorosulfanyl)benzyl)benzamide,
   p-Chloro-N-(4-(trifluoromethyl)benzyl)benzamide, and
   p-Fluoro-N-(4-(trifluoromethyl)benzyl)benzamide.

The composition mentioned above was found to be especially advantageous, concerning the adhesion and viscosity properties and the uptake of a pharmaceutically active agent from the inventive non-aqueous gel composition into the body providing a sustained release of the compound for a period of at least 3 days, preferably at least 5 days, after a single administration at organic tissue.

The invention further provides a syringe, which is a discharging device filled with a non-aqueous gel composition as claimed and as defined above.

In an embodiment of the invention the discharging device is preferably connected to a suitable needle for transtympanic administration, preferably a needle with a diameter of 18 to 27 gauge (Birmingham gauge), more preferable of 20 to 23 gauge, in particular of 20 gauge, suitable to apply small volumes from 20 µL to 200 µL of the inventive non-aqueous gel composition, in particular for transtympanic administration.

In another embodiment of the invention the discharging device is preferably a CycloOlefinPolymer (COP) syringe, which shows the advantage of bearing no risk to have any residual glass particles from the manufacturing process included.

In another embodiment of the invention the discharging device preferably comprises a stopper and a plunger.

Further the discharging device preferably comprises a syringe holder, in particular a plastic or cardboard syringe holder, which is especially useful to avoid any damage of the syringe during transport.

In a further embodiment of the invention the syringe is preferably filled under aseptic conditions with a sterile, inventive non-aqueous gel composition.

With respect to further features and advantages of the syringe, in particular in terms of the non-aqueous gel composition, reference is made in its entirety to the previous description. Finally, the invention provides a non-aqueous gel composition comprising at least one pharmaceutically active agent as claimed and as defined above, for use as a pharmaceutical composition or a medicament, wherein preferably said use is for the prevention or treatment of inner ear diseases, wherein preferably said non-aqueous gel composition is provided for transtympanic administration.

In an embodiment of the invention it is preferred that the non-aqueous gel composition comprising at least one pharmaceutically active agent is administered via transtympanic administration providing a sustained release of the pharmaceutically active agent into the ear, preferably the inner ear, for a period of at least 3 days, preferably at least 5 days, in particular after a single administration. Because of its viscosity properties described above the inventive non-aqueous gel composition is especially useful for a sustained release of a pharmaceutically active agent, especially for transtympanic administration for the treatment of otic disorders including but not limited to induced hearing loss and drug-induced ototoxicity.

In addition, the inventive non-aqueous gel composition advantageously shows a well-regulated viscosity and gel structure especially suitable for administration as droplets. Following transtympanic administration, the composition preferably adheres, in particular in form of a droplet, on the round window membrane or adjuvant mucosa and is not washed away from the middle ear and/or the round window membrane and is therefore providing a sustained release of the pharmaceutically active agent for a period of at least 3 days, preferably at least 5 days, after a single administration at organic tissue, thereby avoiding frequent transtympanic administration into a patients ear.

With respect to further features and advantages of the use, in particular in terms of the non-aqueous gel composition, reference is made in its entirety to the previous description.

Further features and advantages of the invention will become clear from the following examples in conjunction with the subject-matter of the dependent claims. The individual features can be realized either singularly or in combination in one embodiment of the invention. The preferred embodiments merely serve for illustration and better understanding of the invention and are not to be understood as in any way limiting the invention.

### EXAMPLES

### Lab scale trials:

As pharmaceutically active agent (1SR, 2SR, 4RS)-N-(3-(pentafluoro-λ6-sulfaneyl)benzyl)bicyclo [2.2.1]heptane-2-carboxamide (Compound A) or (1S, 2S, 4R)-N-(3-(pentafluoro-λ6-sulfaneyl)benzyl)bicyclo[2.2.1]heptane-2-carboxamide (Compound B) were used for the trials.

A pharmaceutically active agent is grinded in a mortar/pestle system to the appropriate particles size prior to its use.

Afterwards the grinded pharmaceutically active agent is intensively mixed with a surfactant to get almost fully wetted pharmaceutically active agent particles. The mixing is performed in a double jacket glass reactor. Afterwards the pre-warmed (at about 50°C) lipophilic carrier is added stepwise under intensive mixing. The suspension is stirred for an appropriate time to ensure a homogeneous suspension of the pharmaceutically active agent. Finally, the thickener is added carefully under stirring at about 50°C until it is fully dispersed and the non-aqueous gel composition has been formed. The final non-aqueous gel composition is placed in a glass bottle and the included air bubbles are removed under reduced pressure (about 150-80 mbar first, followed by about 18-20 mbar). In case filling is performed, the final non-aqueous gel composition is filled into transparent e.g. luer lock CycloOlefinPolymer or CycloOlefinCopolymer (COP or COC) syringes or glass syringes type I of appropriate size (e.g. 0.5, 1.0, 2.25, 3.0 ml) with the help of a filling system and the stoppers are placed finally on top of the non-aqueous gel composition to close the syringes. The syringes are stored horizontally or vertically under appropriate conditions (e.g. at 2-8°C or ≤ 25°C) prior use.

### Pilot scale trials under sterile conditions:

All operations need to be done in a sterile environment via an isolator system. Before starting the process, the sterile starting materials need to be prepared. In addition, the samples for bioburden must be taken before sterilisation of the materials.

The excipients Labrafil^{®} M 1944 CS and refined castor oil are sterilised by sterile filtration, each with 2 filter capsules in-line. A polyethersulfon (PES) membrane, consisting of one layer with 0.65 µm and 0.2 µm has been identified as optimal but also other membranes maybe used. Filter integrity tests before and after filtration, are performed. Each individual filter is tested. The integrity tests before sterile filtration are carried out with water-wetted membranes. Before sterile filtration takes place, the water is removed. An appropriate pre-flow of the sterile filtered material is discarded.

When sterile filtration is completed, the integrity test of the filter membranes is carried out once more.

In order to facilitate the filtration, Castor oil and Labrafil^{®} M 1944 CS are pre-warmed to about 50-55 °C. Filtration needs to be performed shortly before manufacturing starts.

Depending on the concentration of the active pharmaceutical ingredient (API) in the oleogel, the active pharmaceutical ingredient can be added to the mixture of Castor oil and Labrafil^{®} M 1944 CS and sterile filtrated. If the concentration of the active pharmaceutical ingredient is exceeding the solubilisation limit in the oleogel, the Castor oil and the Labrafil^{®} M 1944 CS need to be sterile filtrated separately and the active pharmaceutical ingredient has to be gamma irradiated and added afterwards in a sterile environment to the sterile filtered mixture of Castor oil and Labrafil^{®} M 1944 CS.

The silicon dioxide is an insoluble solid which needs to be gamma irradiated prior to the manufacturing to get it sterile. The active pharmaceutical ingredient has only to be gamma irradiated in case of high, the solubility limit extending concentrations. The quantities needed for the batch manufacturing are pre-weighed with the accurate masses, that are required for the current batch size, and subsequently gamma irradiated. The containers with the irradiated material are stored until the manufacturing starts.

In case of a solution of the active pharmaceutical ingredient in the oily matrix the following manufacturing steps are applied:
The manufacturing of the oleogel is carried out in a double-jacketed glass reactor. In a first step, Labrafil^{®} M 1944 CS and Castor oil are pre-mixed at about 50-55°C and the active pharmaceutical ingredient is dissolved in this mixture. Afterwards this mixture is sterile filtered into the jacketed glass reactor (solution 1). The reactor is pre-tempered to about 50-55 °C.

Dispersion of the sterile silicon dioxide must be carried out quickly, in order to avoid high viscosity of the dispersion before all silicon dioxide is wetted. After the whole amount of silicon dioxide has been dispersed to solution 1, the homogenisation of the composition is completed using an Ultra-Turrax and further stirring.

The removal of incorporated air, which takes place directly in the glass reactor, is performed afterwards. After connection to a suitable vacuum pump and separating of the reactor and the pump with a sterile air filter, the removal is performed by applying reduced pressure (approx. 25 mbar) over a certain period of time. The pressure reduction needs to be done carefully controlling the rise of air bubbles in order to prevent delay in boiling.

Finally, the glass reactor is connected to a suitable filling pump. Filling of the syringes is performed and finally stoppering takes place. The syringes are placed back into their nests and transferred out of the isolator.

The syringes are labelled with a transparent label with two marks that support accurate application of the intended small volume (e.g.100 µl).

### Examples for non-aqueous gel compositions

Several lab scale batches have been produced in accordance to the general procedure described above with and without addition of a pharmaceutically active agent. The following tables 1-3 give an overview of some relevant batches:

**Table 1: Formulation batches**

| **Material** | **F1** | **F2** | **F3** | **F4** | **F5** | **F6** |
|---|---|---|---|---|---|---|
| Compound A | - | - | - | - | - | - |
| Castor oil, native | 95.0 | 92.0 | 91.0 | 88.0 | 94.0 | 93.0 |
| Oleoyl Polyoxy-6-glyceride (Labrafil^{®} M 1944 CS) | 2.50 | 4.00 | 4.50 | 6.00 | 3.00 | 3.50 |
| Silicon dioxide (Aerosil 200 PH) | 2.50 | 4.00 | 4.50 | 6.00 | 3.00 | 3.50 |

**Table 2: Formulation batches**

| **Material** | **F6 (1)** | **F7a** | **F7b** | **F9a** | **F9b** | **F9c** | **F9d** | **F9e** | **F9f** | **F9g** | **F9h** |
|---|---|---|---|---|---|---|---|---|---|---|---|
| Compound A | - | | 0.60 | - | - | - | - | - | - | 0.60 | 0.60 |
| Castor oil, native | 93.0 | 93.0 | 91.4 | 93.5 | 93.25 | 93.0 | 92.75 | 92.5 | 92.25 | 90.90 | 89.90 |
| Oleoyl Polyoxy-6-glyceride (Labrafil^{®} M 1944 CS) | 3.50 | - | 4.50 | - | - | - | - | - | - | - | - |
| Glycerol Monooleate (Capmul^{®} GMO 50) | - | 3.50 | - | 4.50 | 4.50 | 4.50 | 4.50 | 4.50 | 4.50 | 4.50 | 4.50 |
| Silicon dioxide (Aerosil 200 PH) | 3.50 | 3.50 | 3.50 | 2.0 | 2.25 | 2.50 | 2.75 | 3.00 | 3.25 | 4.00 | 5.00 |

**Table 3: Formulation batches**

| **Material** | **F10a** | **F10b** | **F10c** | **F10d** | **F10e** | **F10f** | **F11a** | **F11b** | **F11c** |
|---|---|---|---|---|---|---|---|---|---|
| Compound A | - | - | - | - | - | - | 6.00 | 6.00 | 6.00 |
| Castor oil, native | 92.00 | 91.00 | 90.00 | 91.75 | 90.75 | 89.75 | 86.50 | 85.50 | 85.50 |
| Oleoyl Polyoxy-6-glyceride (Labrafil^{®} M 1944 CS) | - | - | - | - | - | - | - | - | - |
| Glycerol Monooleate (Capmul^{®} GMO 50) | 4.50 | 4.50 | 4.50 | 4.50 | 4.50 | 4.50 | 4.50 | 4.50 | 4.50 |
| Silicon dioxide (Aerosil 200 PH) | 2.50 | 2.50 | 2.50 | 2.75 | 2.75 | 2.75 | 3.00 | 4.00 | 5.00 |
| Silicon dioxide (SilSol 6035) | 1.00 | 2.00 | 3.00 | 1.00 | 2.00 | 3.00 | - | - | - |

Capmul^{®} is an acylglycerol. Capmul^{®} GMO 50 is a glycerol monooleate. Capmul^{®} is a registered trademark of ABITEC. The Capmul^{®} series includes Capmul^{®} GMO 50, Capmul^{®} 708G (glyceryl monocaprylate), Capmul^{®} MCM (medium chain mono- and diglycerides) and Capmul^{®} MCM C8 (glyceryl monocaprylate), Capmul^{®} PG-8 (propylene glycol monocaprylate), Capmul^{®} PG-12 (propylene glycol monolaurate), Capmul^{®} PG-2L (propylene glycol dilaurate), and Capmul^{®} S12L (sodium lauroyl lactylate).

SILSOL^{®} is a 6035 mesoporous silica dioxide and a product of W. R. Grace & Co.-Conn.

In another embodiment, lab scale batches with the final selected composition have been produced as described above. As representative examples two batches are described:

### Lab scale batch A

Batch size: about 250 g
Strength: 0.6 % of active ingredient Compound B
Composition (table 4)

**Table 4 Lab scale batch A**

| **Material** | **Batch** | **Concentration [%]** | **Quantity [g]** |
|---|---|---|---|
| Compound B | CC-5875.0-09 | 0.60 | 1.500 |
| Castor oil, refined (Gustav Heess) | 150004 | 93.9 | 234.8 |
| Silicon dioxide (Aerosil 200 PH) | 17003144 | 2.00 | 5.000 |
| Oleoyl Polyoxy-6-glyceride (Labrafil^{®} M 1944 CS) | 162835 | 3.50 | 8.750 |
| **Sum** | | 100.0 | 250.0 |

### Lab scale batch B

Batch size: about 250 g
Strength: 6.0 % of active ingredient Compund B
Composition (table 5)

**Table 5 Lab scale batch B**

| **Material** | **Batch** | **Concentration [%]** | **Quantity [g]** |
|---|---|---|---|
| Compound B | CC-5875.0-09 | 6.00 | 15.00 |
| Castor oil, refined (Gustav Heess) | 150004 | 88.5 | 221.3 |
| Silicon dioxide (Aerosil 200 PH) | 17003144 | 2.00 | 5.000 |
| Oleoyl Polyoxy-6-glyceride (Labrafil^{®} M 1944 CS) | 162835 | 3.50 | 8.750 |
| **Sum** | | 100.0 | 250.0 |

Pilot scale batches under sterile conditions have been produced as described above. As an example two representative batches are described:

### Pilot scale batch A

Batch size: about 2200g
Strength: 0.6 % of active ingredient Compound B
Composition (table 6)

**Table 6 Pilot scale batch A**

| **Pos. No.** | **Material** | **Function** | **Concentration [%]** | **Quantitative Composition [g]** |
|---|---|---|---|---|
| 01 | Compound B | API | 0.6 | 13.200 |
| 02 | Castor oil, refined | Lipophilic carrier | 93.9 | 2065.8 |
| 03 | Labrafil^{®} M 1944 CS (Oleoyl macrogolgylcerides) | Surfactant | 3.5 | 77.000 |
| 04 | Aerosil 200 PH (Silica, colloidal anhydrous) | Gelling agent | 2.0 | 44.000 |
| **Sum** | | | **100** | **2200.0** |

### Pilot scale batch B

Batch size: about 2200g
Strength: 6.0 % of active ingredient Compound B
Composition (table 7)

**Table 7 Pilot scale batch B**

| **Pos. No.** | **Material** | **Function** | **Concentration [%]** | **Quantitative Composition [g]** |
|---|---|---|---|---|
| 01 | Compound B | API | 6.00 | 132.00 |
| 02 | Castor oil, refined | Lipophilic carrier | 88.5 | 1947.0 |
| 03 | Labrafil^{®} M 1944 CS (Oleoyl macrogolgylcerides) | Surfactant | 3.50 | 77.000 |
| 04 | Aerosil 200 PH | Gelling agent | 2.00 | 44.000 |
| | (Silica, colloidal anhydrous) | | | |
| **Sum** | | | **100** | **2200.0** |

### Rheological and syringeability measurements

Viscosities are measured with a Brookfield RST Cone Plate Rheometer. A shear speed of 500 1/s with constant rotation can be used. A suitable measurement time is 120 seconds. As measuring temperatures 25°C and 37°C are used.

For preparing the sample and the measurement, the rheometer is tempered to the desired measurement temperature via thermostat. Then, the sample (0.6 ml) is placed dropwise on the sample plate via scaled pipette, Eppendorf Pipette or a suitable syringe to achieve a complete filling of the measuring gap of the rheometer. After removing excess sample material, the sample is also tempered to the desired temperature and measured.

In a further embodiment, some of the formulation batches as listed above have been tested on adhesion properties, viscosity and syringeability:

**Table 8: syringeability test (visual) and adhesion test**

| **Parameter** | **F1** | **F2** | **F3** | **F4** | **F5** | **F6** |
|---|---|---|---|---|---|---|
| Syringeability = application composition from syringe with 23G needle (at about 25°C) | Slight resistance during application | Slight resistance during application | Strong resistance during application | Not possible | Slight resistance during application | Slight resistance during application |
| Run-off tendency of droplet in adhesion test | High | Low | Low | Low | Moderate | Low |

The related viscosity values for F1-F6 are:

**Table 9: viscosity values at 25°C and 37°C**

| **Parameter** | **F1** | **F2** | **F3** | **F4** | **F5** | **F6** |
|---|---|---|---|---|---|---|
| Average viscosity at 25°C [mPas] | 1427 | 1845 | 2207 | n.a. | 1584 | 1963 |
| Average viscosity at 37°C [mPas] | 578 | 908 | 1111 | n.a. | 725 | 842 |

In another embodiment, in particular the non-aqueous gel composition containing castor oil, oleoyl macgrogolglycerides (Labrafil^{®} M 1944 CS) and silicon dioxide (Aerosil 200 PH also known as Aerosil^{®} Pharma, product of Evonik Operations GmbH) have been subject to the syringeability and adhesion test:

**Table 10: syringeability values**

| **Batch** | **Compound B [%]** | **Castor oil [%]** | **Labrafil^{®} 1944 [%]** | **Aerosil 200 PH [%]** | **Test with 20G needle at 25°C** | **Test with 20G needle at 37°C** |
|---|---|---|---|---|---|---|
| 1 | 6.00 | 87.0 | 3.50 | 3.50 | 16 sec | 12 sec |
| 2 | 6.00 | 88.0 | 3.50 | 2.50 | 11 sec | 9 sec |
| 3 | 6.00 | 88.5 | 3.50 | 2.00 | 10 sec | 7 sec |

### Particle size distribution (PSD) of non-aqueous gel composition containing Compound B

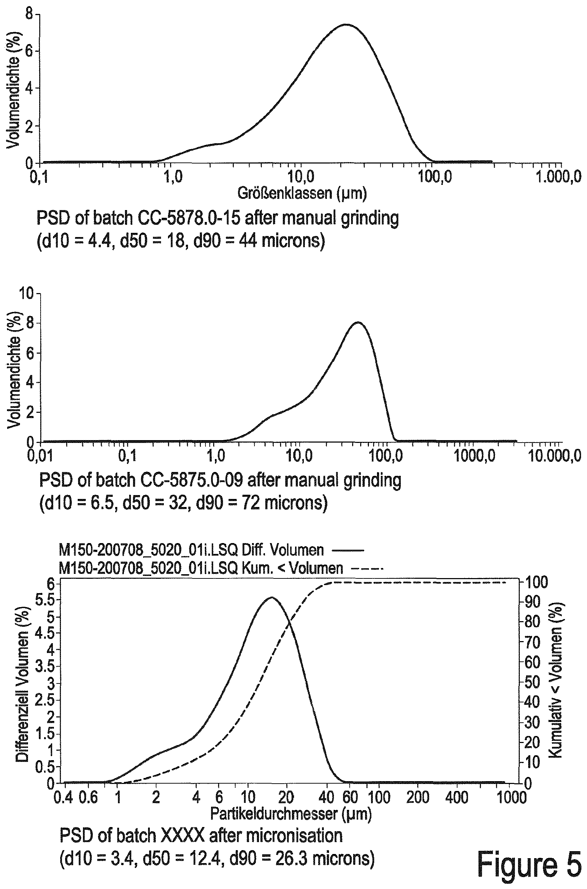

In some embodiments, the PSD (Particle Size Distribution) of the pharmaceutically active agent was measured with a Malvern MasterSizer 2000 or 3000 (in water as dispersion medium, stirrer speed 1500 rpm, Mie presentation) to give the following results (table 11). The PSD of the active ingredient remained unchanged in the non-aqueous gel composition. Some of the grinded batches are reported with the full PSD profile in figure 5:

**Table 11: PSD of Compound B batches after grinding**

| **Batch** | **D10 [µm]** | **D50 [µm]** | **D90 [µm]** |
|---|---|---|---|
| 1 | 3.93 | 20.6 | 57.5 |
| 2 | 6.51 | 31.7 | 71.7 |
| 3 | 3.93 | 20.6 | 57.5 |
| 4 | 6.51 | 31.7 | 72.4 |
| 5 | 6.95 | 32.8 | 71.4 |

Several lab scale batches have been produced in accordance to the general procedure described above with and without addition of the oleoyl macrogolglyceride Labrafil^{®} and with and without the addition of a pharmaceutically active agent (API). The following tables 11a and 11b give an overview of some relevant batches. Tables 11a and 11b show that Labrafil^{®} in the inventive non-aqueous gel composition has the advantage over Capmul^{®} to result in a composition with better syringeability and a better run-off tendency.

The batches F6, F2, F7b and 6 comprising Labrafil^{®} resulted in compositions with slight or moderate resistance during application with a 23G needle (at about 25°C) and are therefore easy to dispense from, and/or drawn into, a syringe. In comparison the batches F7a, F10al, F8b and 7 comprising Capmul^{®} resulted in compositions with no resistance during application with a 23G needle (at about 25°C) and are therefore less suitable to apply during use since they are dripping out of the syringe without applying much pressure to the syringe.

Similar results were obtained when testing the run-off tendency of droplets in an adhesion test which simulates the adhesion of the composition at organic tissues. While the batches F6, F2, F7b and 6 comprising Labrafil^{®} resulted in compositions with low run-off tendency and are therefore suitable for an adhesion to organic tissues. The batches F7a, F10a, F8b and 7 comprising Capmul^{®} resulted in compositions with a high or moderate run-off tendency and are therefore less suitable for an adhesion to organic tissues in particular for a period of at least 3 days, after a single administration.

For the adhesion test droplets of each composition were placed on a petri dish. The petri dish was then placed upright to recognize possible run-off effects of the droplets. Additionally, further droplets were placed on a petri dish which afterwards was filled with water to recognize possible detaching effect of the droplet.

**Table 11a Syringeability test (visual) and adhesion test without API**

| **Material** | **F6** | **F7a** | **F2** | **F10al** |
|---|---|---|---|---|
| Castor oil, refined | 93 | 93 | 92 | 92 |
| Oleoyl Polyoxy-6-glyceride (Labrafil^{®} M 1944 CS) | 3,5 | - | 4 | - |
| Glycerol Monooleate (Capmul^{®} GMO 50) | - | 3,5 | - | 4 |
| Aerosil 200 PH (Silica, colloidal anhydrous) | 3,5 | 3,5 | 4 | 4 |
| API | - | - | - | - |
| **Parameter** | **F6** | **F7a** | **F2** | **F10al** |
| Syringeability = application composition from syringe with 23G needle (at about 25°C) | Slight resistance during application | No resistance during application | Slight resistance during application | Almost no resistance during application |
| Run-off tendency of droplet in adhesion test | Low | High | Low | High |

**Table 11b Syringeability test (visual) and adhesion test**

| **Material** | **F7b** | **F8b** | **Batch 6** | **Batch 7** |
|---|---|---|---|---|
| Castor oil, refined | 91,4 | 91,4 | 86,5 | 86,5 |
| Oleoyl Polyoxy-6-glyceride (Labrafil^{®} M 1944 CS) | 4,5 | - | 4,5 | - |
| Glycerol Monooleate (Capmul^{®} GMO 50) | - | 4,5 | - | 4,5 |
| Aerosil 200 PH (Silica, colloidal anhydrous) | 3,5 | 3,5 | 3,5 | 3,5 |
| API (Compound A) | 0,6 | 0,6 | 6 | 6 |

| **Parameter** | **F7b** | **F8b** | **Batch 6** | **Batch 7** |
|---|---|---|---|---|
| Syringeability = application composition from syringe with 23G needle (at about 25°C) | Slight resistance during application | Certain resistance during application | Moderate resistance during application | Certain resistance during application |
| Run-off tendency of droplet in adhesion test | Low | Moderate | Low | Moderate |

### Sterilisation

The following conditions have been used for the silicon dioxide:
*Source:* ⁶⁰Co
*Packaging:* Brown glass bottles or HDPE (High Density Poly Ethylene) bottles with irradiation resistant caps packaged in dry ice
*Irradiation dose:* 25-50 kGy (standard range)

For the sterile filtrations of the castor oil and Labrafil^{®} or Castor oil/Labrafil mixtures PES filters have been used. For the aseptic environment an Isolator with ISO 5 class qualification has been used. Sterility of the silicon dioxide and the final product have been proven with validated Ph.Eur. (European Pharmacopoeia) methods.

### Impurity profile of the active ingredient before and after gamma-irradiation

In some embodiments, the impurity profile of the *Compound B* has been investigated by HPLC prior and after gamma irradiation proving that the gamma irradiation (standard conditions) only minor effects the quality of the active ingredient if dry ice is used for cooling.

### ACOU-085, lot CC-5875.0-09.00, [Area % impurities]

**Table 12: Impurity profile of Compound B prior and after gamma irradiation**

| | **Initial API** | **Gamma irradiated at 49.6 KGy** | **Gamma irradiated at 49.6 KGy under dry ice** |
|---|---|---|---|
| Imp. 1. rel.tR= 0.22 | n.a. | 0.11 | 0.23 |
| Imp. 2. rel.tR= 0.37 | n.a. | 0.11 | 0.03 |
| Imp. 3. rel.tR= 0.48 | 0.13 | 0.24 | 0.12 |
| Imp. 4. rel.tR= 0.52 | <0.05 | 0.12 | 0.12 |
| Imp. 5. rel.tR= 0.54 | n.a. | 0.08 | 0.05 |
| Imp. 6. rel.tR= 0.58 | n.a. | 0.14 | 0.15 |
| Imp. 7. rel.tR= 0.71 | <0.05 | 0.39 | 0.34 |
| *Imp. 8. rel.tR= 0.95 | **3.48** | 3.29 | 3.35 |
| Imp. 9. rel.tR= 1.27 | n.a. | 0.32 | 0.20 |
| Imp. 10. rel.tR= 1.80 | n.a. | 0.06 | n.a. |
| **Sum of impurities** | **3.61** | **4.86** | **4.59** |
| **impurity Increase compared to initial** | | **+ 1.25** | **+ 0.98** |

### Dissolution profiles

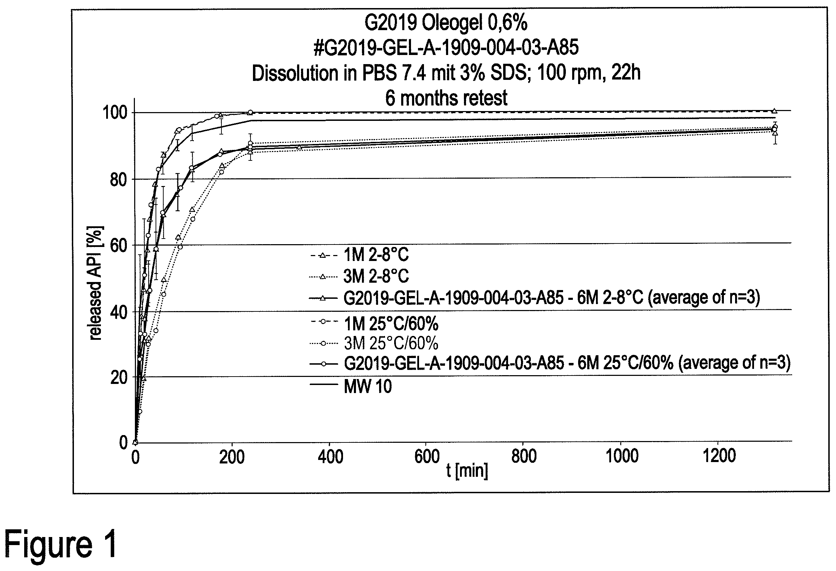

In further embodiments the in-vitro dissolution has been tested with a Sotax T7 smart US paddle system at 37°C, with 100 rpm at pH 7.4 (Phosphate-buffered Saline (PBS)) with 3 % sodium dodecyl sulphate (SDS). A defined quantity of different non-aqueous gel compositions is distributed homogeneous on a watch glass (diameter 8 cm) and the loaded watch glass placed at the bottom of the dissolution vessel. The results are reported exemplarily in figures 1, 2 and 9 or tabulated below for stability samples.

### Stability

In another embodiment, the stability of the inventive non-aqueous gel composition has been investigated. The results are reported exemplarily for the 0.6% non-aqueous gel composition formulation described in Table 4 and Table 5. The results shown prove the excellent stability over the investigated interval at 2-8°C and 25°C/60 % relative humidity.

### Pharmacokinetic profiles

### Studies in guinea pigs

### Application

transtympanic as single dose of 100 µL

### Animal Specification

Species: Dunkin Hartley Guinea Pig
Sex: female

### Experimental groups and doses

Group: 3 per time point
Dose: 100 µL 0.6% non-aqueous gel composition (described in Table 4 and Table 5) or 100 µL 6% non-aqueous gel composition (described in Table 4 and Table 5) / animal / ear

For a better understanding of what has been disclosed, some figures are attached which schematically or graphically and solely by way of non-limiting example show a practical case of embodiment of the present invention.
Figure 1 graphically shows in-vitro dissolution data (apparatus US Paddle II, 37°C) of a non-aqueous gel composition containing 0.6% Compound B (described in Table 4 and Table 5) (initial, 3 months and 6 months).
Figure 2 graphically shows in-vitro dissolution data (apparatus US Paddle II, 37°C) of a non-aqueous gel composition containing 6% Compound B (described in Table 4 and Table 5) (initial and 3 months).
Figure 3 graphically shows pharmaceutically active agent levels of a pharmacokinetic (pk) study in guinea pigs of a non-aqueous gel composition containing 6% Compound B (described in Table 4 and Table 5).
Figure 4 graphically shows pharmaceutically active agent levels (inner ear tissue, mean values) of a study in guinea pigs with a non-aqueous gel composition containing 6% Compound B (described in Table 4 and Table 5).
Figure 5 graphically shows examples for Particle Size Distribution (PSD) of Compound B batches used in the inventive non-aqueous gel composition (Master Sizer results).
Figure 6 shows stability data of a non-aqueous gel composition containing 0.6 % Compound B in a syringe stored over 6 months at 2-8°C.
Figure 7 shows stability data of a non-aqueous gel composition containing 0.6 % Compound B in a syringe stored over 6 months at 25°C/60% r.h. (relative humidity)
Figure 8 graphically shows the viscosity of non-aqueous gel compositions containing 0.6% and 6% Compound B with shear stress.
Figure 9 graphically shows an in vitro dissolution profile (apparatus US Paddle II, 37°C) of a non-aqueous gel formulations containing 0.6% Compound B.
Figure 10 graphically shows a viscosity measurement of F1 - F3, F5 and F6 (here listed as V1, V2, V3, V5, V6) at 25°C.
Figure 11 graphically shows a viscosity measurement of F1 - F3, F5 and F6 (here listed as V1, V2, V3, V5, V6) at 37°C.

Figure 1 is explained as follows: Figure 1 demonstrates the constant release profile of the non-aqueous gel composition containing 0.6 % Compound B over 22 hours at 37°C in a US Paddle II method after a storage at 2-8°C and 25°C/60r.h. over 1 month (1 M) and 3 months (3 M). Single and mean values with n=3 are presented in comparison to the initial release profile (MWt0).

"Released API" means "released Active Pharmaceutical Ingredient".

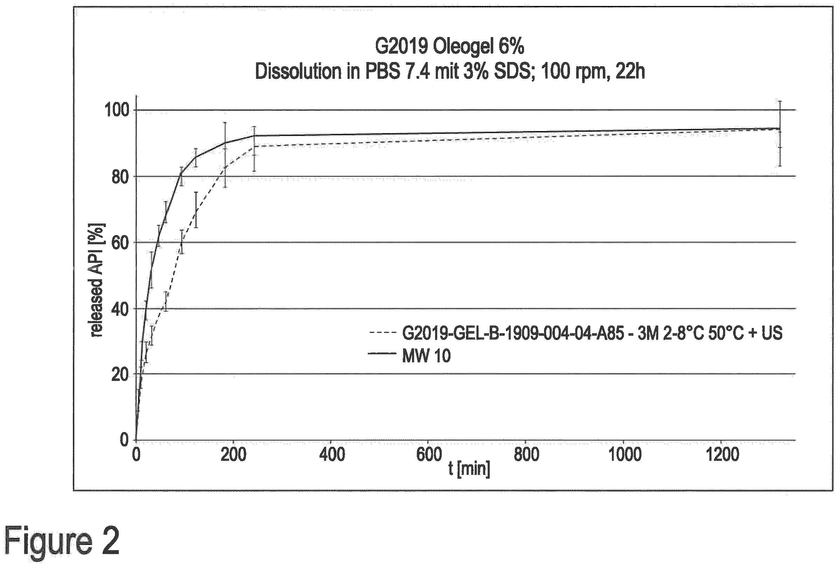

Figure 2 is explained as follows: Figure 2 demonstrates the effect of a thermal treatment at 50°C over 2 hours followed by an ultrasonic (US) treatment over 15 min. (possible re-homogenisation) for the non-aqueous gel composition containing 6 % Compound B stored at 2-8°C over a period of 3 months (3 M) and the impact on the dissolution profile at 37°C in a US-Paddle apparatus in comparison to the initial release profile (MWt0).

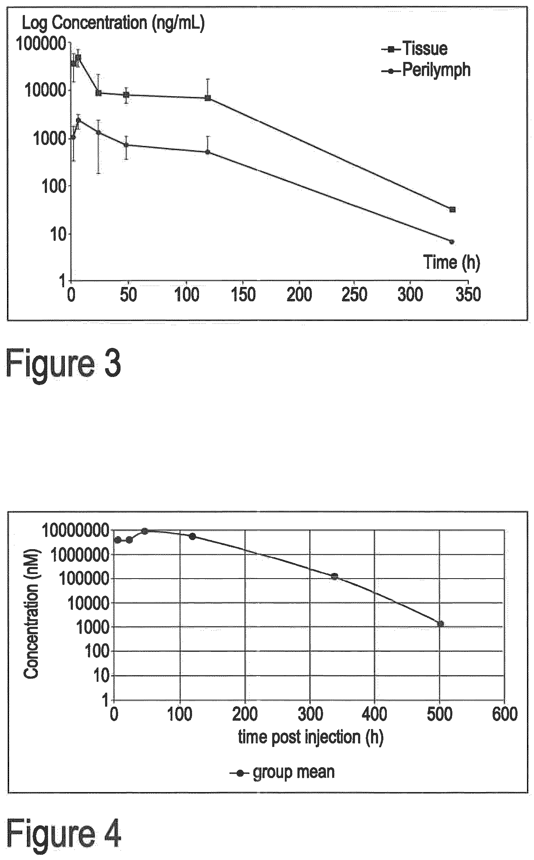

Figure 3 is explained as follows: Figure 3 shows pharmaceutically active agent concentration levels in ng/ml of a pk study in guinea pigs in the tissue and perilymph of a non-aqueous gel composition containing 6% Compound B demonstrating the sustained release effect over a period of > 150 hours.

Figure 4 is explained as follows: Figure 4 shows pharmaceutically active agent concentration levels in nM of a PK study in guinea pigs in the tissue and perilymph of a non-aqueous gel composition containing 6% Compound B as mean values demonstrating the sustained release effect over a period of > 150 hours.

Figure 5 is explained as follows: Figure 5 graphically shows examples for the Particle Size Distribution (PSD) of Compound B batches used in the inventive non-aqueous gel composition (MasterSizer results) with reported d10 values of 4.4 respective 6.5 microns, d50 values with 18 respective 32 microns and d90 values with 44 respective 72 microns for manual grinded samples (upper and middle illustration) and a d10 value of 3.4, a d50 value of 12.4 and a d90 value of 26.3 microns for a micronized sample using a jet mill for micronisation (lower illustration).

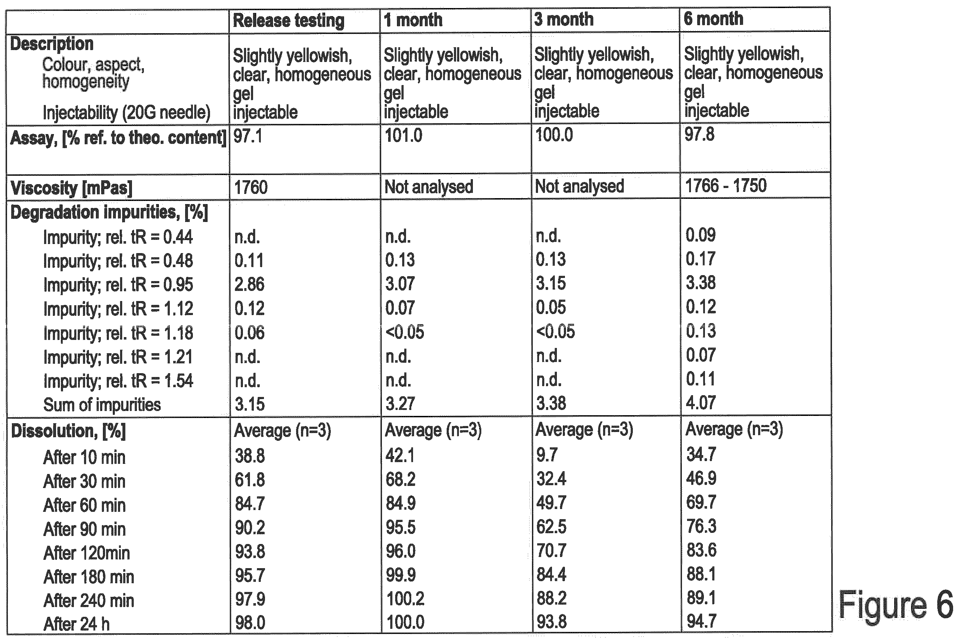

Figure 6 is explained as follows: Figure 6 shows the stability data of a non-aqueous gel composition containing 0.6 % Compound B in a syringe stored over 6 months at 2-8°C and analysed on the presented parameters description, assay (by HPLC), viscosity, degradation (by HPLC) and dissolution at 37°C (US Paddle Typ II) demonstrating the good stability of the non - aqueous gel over the investigated period.

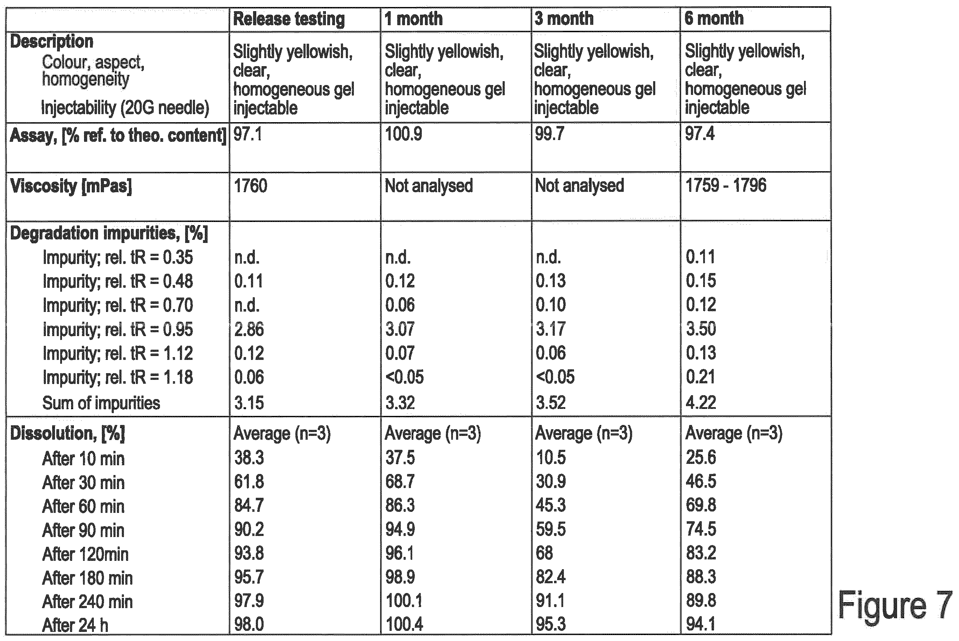

Figure 7 is explained as follows: Figure 7 shows the stability data of a non-aqueous gel composition containing 0.6 % Compound B in a syringe stored over 6 months at 25°C/60%r.h. and analysed on the presented parameters description, assay (by HPLC), viscosity, degradation (by HPLC) and dissolution at 37°C (US Paddle Typ II) demonstrating the good stability of the non -aqueous gel over the investigated period.

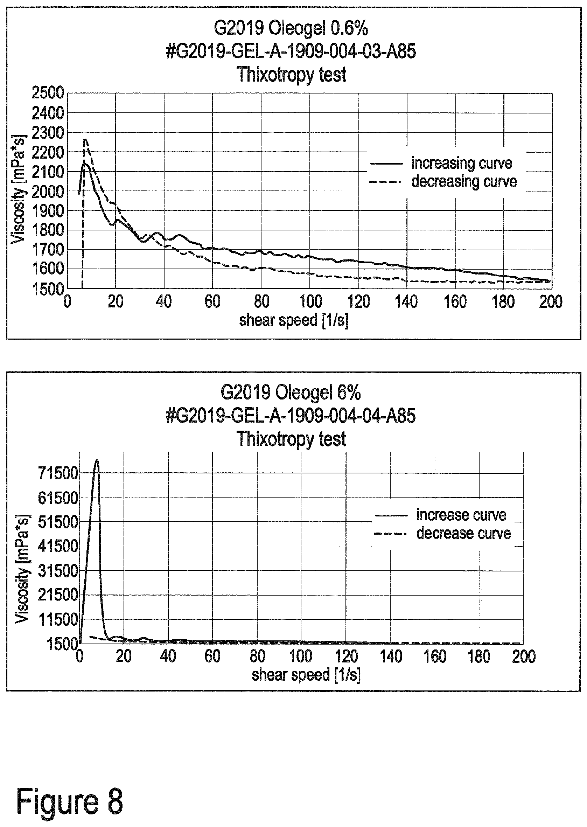

Figure 8 is explained as follows: Figure 8 (upper graphic) graphically shows the viscosity of non-aqueous gel compositions containing 0.6% Compound B at a temperature between 20°C and 25°C with shear stress, demonstrating that the stress increasing and stress decreasing curve are almost identical and therefore proving that from the initial values of about 2100-2200 mPa*s the viscosity is decreasing to a steady state level of about 1500 -1600 mPa*s with increasing shear speed (stress) as typical for a thixotropic medium and a reversible effect.

Figure 8 (lower graphic) graphically shows the viscosity of non-aqueous gel compositions containing 6% Compound B at a temperature between 20°C and 25°C with shear stress, proving that from the initial value of about 71500 mPa*s the viscosity is decreasing to a steady state level of about 1500 mPa*s with increasing shear speed (stress) as typical for a thixotropic medium, but maintaining the shear effect over longer periods and staying at low viscosity levels (due to the higher loading with Compound B) when decreasing the shear speed.

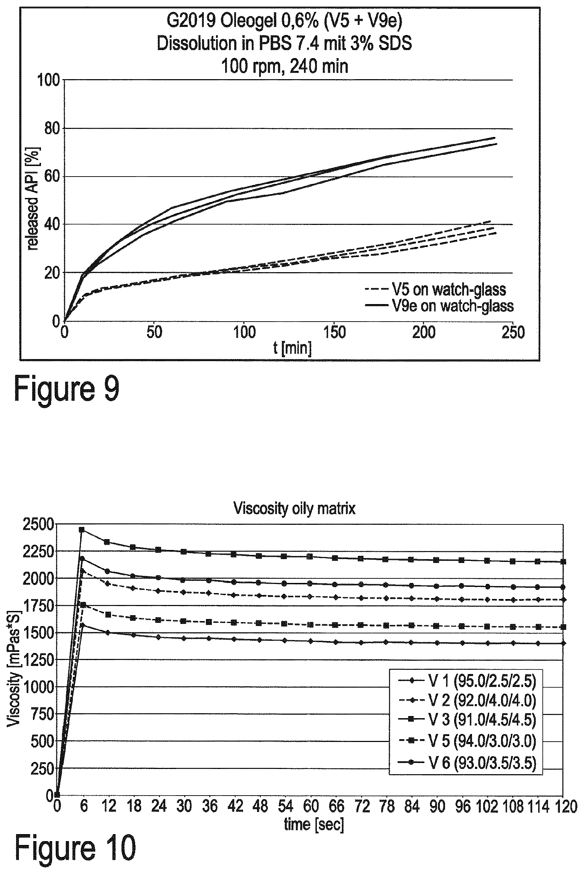

Figure 9 is explained as follows: Figure 9 graphically shows an in vitro dissolution profile (apparatus US Paddle II, 37°C) of two non-aqueous gel formulations containing 0.6% Compound B and the effect of the change of the surfactant in the composition on the release profile (refer to Tables 1 and 2; V5 identical to F5 and V9e identical to F9e, as source batches).

Figure 10 is explained as follows: Figure 10 graphically shows a viscosity measurement of F1, F2, F3, F5 and F6 from Table 1(here listed as V1, V2, V3, V5 and V6 at 25°C. Composition of F1, F2, F3, F5 and F6 identical to V1, V2, V3, V5 and V6. Assignment was changed as this Figure is related to a new experiment. The graph shows the increase of the viscosity in dependency on the composition of the non-aqueous gel.

In line with the earlier explanation the viscosity measurements were made with the Brookfield RST-CPS (Cone Plate) Rheometer at a temperature of 25°C. A shear speed of 500 1/s was used.

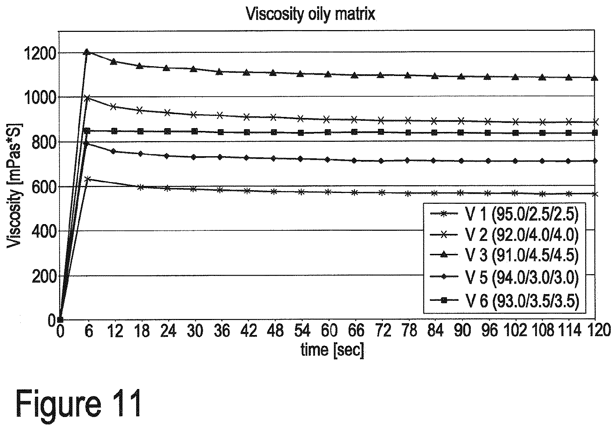

Figure 11 is explained as follows: Figure 11 graphically shows a viscosity measurement of F1, F2, F3, F5 and F6 (here listed as V1, V2, V3, V5 and V6 at 37°C. Composition of F1, F2, F3, F5 and F6 identical to V1, V2, V3, V5 and V6. Assignment was changed as this Figure is related to a new experiment. The graph shows the increase of the viscosity in dependency on the composition of the non-aqueous gel. The same equipment and conditions (but at 37°C), as explained above with Figure 10 were used.

## Claims

1. Non-aqueous gel composition for transtympanic administration comprising
- castor oil, present in an amount from 85 to 95 % by weight, based on the total weight of the composition,
- Labrafil^{®}, present in an amount from 2 to 6 % by weight, based on the total weight of the composition,
- at least one thickener, preferably silicon dioxide, more preferably colloidal silicon dioxide, present in an amount from 1 to 6 % by weight, based on the total weight of the composition, and wherein
the viscosity of the composition at a temperature of 25°C is between 1400 and 2400 mPas, preferably between 1400 and 1800 mPas,
the non-aqueous gel composition further comprises at least one pharmaceutically active agent, having a mean particle size of 0,01 to 100 µm,
wherein the at least one pharmaceutically active agent has the formula I: 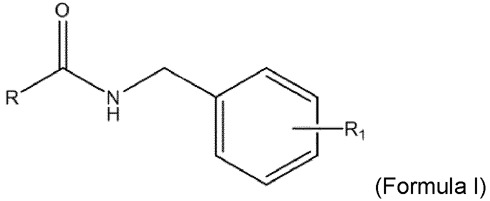 wherein
- R is a unsubstituted cycloalkyl group, in particular bicycloalkyl group,
a unsubstituted or substituted phenyl group, or a unsubstituted or substituted thienyl group, wherein said substituted thienyl group or phenyl group is substituted with at least one halogen, preferably at least one F-atom or at least one Cl-atom, and
- R₁ is F, SF₅, CF₃ or OCF₃.

2. Non-aqueous gel composition according to claim 1, wherein the at least one pharmaceutically active agent is present in an amount from
0,05 to 10 % by weight, preferably from 0,1 to 10 % by weight, more preferably from 0,5 to 5 % by weight, based on the total weight of the composition.

3. Non-aqueous gel composition according to claim 1 or 2, wherein the at least one pharmaceutically active agent has a mean particle size of 5 to 80 µm, preferably of 20 to 50 µm.

4. Non-aqueous gel composition according to anyone of the preceding claims, wherein the at least one pharmaceutically active agent has a (low) solubility in water at room temperature of < 1 mg/mL, preferably < 0,1 mg/mL.

5. Non-aqueous gel composition according to anyone of the preceding claims, wherein the at least one pharmaceutically active agent is selected from a group comprising
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide, and
p-Fluoro-N-(4-(trifluoromethyl)benzyl)benzamide.

6. Non-aqueous gel composition according to claim 5, wherein:
- castor oil is present in an amount from 85 to 95 % by weight, based on the total weight of the composition,
- Labrafil^{®}, is present in an amount from 2 to 6 % by weight, based on the total weight of the composition,
- at least one thickener, preferably silicon dioxide, more preferably colloidal silicon dioxide, present in an amount from 1 to 6 % by weight, based on the total weight of the composition,
- the viscosity of the composition at a temperature of 25°C is between 1400 and 2400 mPas, preferably between 1400 and 1800 mPas,
- the non-aqueous gel composition further comprises at least one pharmaceutically active agent, having a mean particle size of 0,01 to 100 µm and
- the at least one pharmaceutically active agent is selected from a group comprising
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluormethyl)benzyl)bicyclo[2.2. 1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide, and
p-Fluoro-N-(4-(trifluoromethyl)benzyl)benzamide.

7. Non-aqueous gel composition according to claim 5 consisting of:
- castor oil present in an amount from 85 to 95 % by weight, based on the total weight of the composition,
- Labrafil^{®}, present in an amount from 2 to 6 % by weight, based on the total weight of the composition,
- at least one thickener, preferably silicon dioxide, more preferably colloidal silicon dioxide, present in an amount from 1 to 6 % by weight, based on the total weight of the composition,
- at least one pharmaceutically active agent, having a mean particle size of 0,01 to 100 µm and
- the at least one pharmaceutically active agent is selected from a group comprising
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluormethyl)benzyl)bicyclo[2.2.1]heptane-2-carboxamide,
(1R,2R,4S)-rel-N-(3-(trifluoromethoxy)benzyl)bicyclo[2.2.1]heptane-2-carboxamide,
(1S,2S,4R)-N-(3-(trifluoromethyl)benzyl)bicyclo[2.2.1]heptane-2-carboxamide,
(1S,2S,4R)-N-(3-(trifluoromethoxy)benzyl)bicyclo[2.2.1]heptane-2-carboxamide,
p-Chloro-N-(4-trifluoromethoxy)benzyl)benzamide,
p-Chloro-N-(4-(pentafluorosulfanyl)benzyl)benzamide,
p-Fluoro-N-(4-trifluoromethoxy)benzyl)benzamide,
p-Fluoro-N-(4-(pentafluorosulfanyl)benzyl)benzamide,
p-Chloro-N-(4-(trifluoromethyl)benzyl)benzamide, and
p-Fluoro-N-(4-(trifluoromethyl)benzyl)benzamide, wherein
- the viscosity of the composition at a temperature of 25°C is between 1400 and 2400 mPas, preferably between 1400 and 1800 mPas.

8. A syringe, wherein the syringe is filled with a non-aqueous gel composition according to anyone of claims 1 to 7.

9. Non-aqueous gel composition according to anyone of claims 1 to 5, for use as a pharmaceutical composition or a medicament, wherein said use is for the prevention or treatment of inner ear diseases, wherein preferably said non-aqueous gel composition is provided for a transtympanic administration.

## Patentansprüche

1. Nichtwässrige Gelzusammensetzung zur transtympanischen Verabreichung, umfassend
- Rizinusöl, das in einer Menge von 85 bis 95 Gew.-%, bezogen auf das Gesamtgewicht der Zusammensetzung, vorliegt,
- Labrafil^{®}, das in einer Menge von 2 bis 6 Gew.-%, bezogen auf das Gesamtgewicht der Zusammensetzung, vorliegt,
- mindestens ein Verdickungsmittel, vorzugsweise Siliciumdioxid, weiter bevorzugt kolloidales Siliciumdioxid, das in einer Menge von 1 bis 6 Gew.-%, bezogen auf das Gesamtgewicht der Zusammensetzung, vorliegt, und wobei
die Viskosität der Zusammensetzung bei einer Temperatur von 25 °C zwischen 1400 und 2400 mPas, vorzugsweise zwischen 1400 und 1800 mPas, liegt,
wobei die nichtwässrige Gelzusammensetzung ferner mindestens einen pharmazeutischen Wirkstoff mit einer mittleren Teilchengröße von 0,01 bis 100 µm umfasst, wobei der mindestens eine pharmazeutische Wirkstoff die Formel I aufweist: 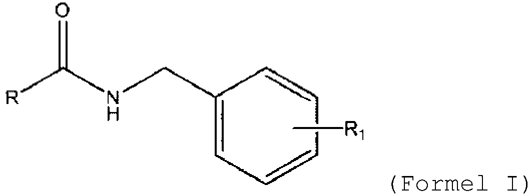
wobei
- R für eine unsubstituierte Cycloalkylgruppe, insbesondere Bicycloalkylgruppe,
eine unsubstituierte oder substituierte Phenylgruppe oder eine unsubstituierte oder substituierte Thienylgruppe steht, wobei die substituierte Thienylgruppe oder Phenylgruppe durch mindestens ein Halogen, vorzugsweise mindestens ein F-Atom oder mindestens ein Cl-Atom, substituiert ist, und
- R₁ für F, SF₅, CF₃ oder OCF₃ steht.

2. Nichtwässrige Gelzusammensetzung nach Anspruch 1, wobei der mindestens eine pharmazeutische Wirkstoff in einer Menge von
0,05 bis 10 Gew.-%, vorzugsweise 0,1 bis 10 Gew.-%, weiter bevorzugt 0,5 bis 5 Gew.-%, bezogen auf das Gesamtgewicht der Zusammensetzung, vorliegt.

3. Nichtwässrige Gelzusammensetzung nach Anspruch 1 oder 2, wobei der mindestens eine pharmazeutische Wirkstoff eine mittlere Teilchengröße von 5 bis 80 µm, vorzugsweise von 20 bis 50 µm, aufweist.

4. Nichtwässrige Gelzusammensetzung nach einem der vorhergehenden Ansprüche, wobei der mindestens eine pharmazeutische Wirkstoff eine (geringe) Löslichkeit in Wasser bei Raumtemperatur von < 1 mg/ml, vorzugsweise < 0,1 mg/ml, aufweist.

5. Nichtwässrige Gelzusammensetzung nach einem der vorhergehenden Ansprüche, wobei der mindestens eine pharmazeutische Wirkstoff ausgewählt ist aus einer Gruppe umfassend
(1R,2R,4S)-rel-N-(3-(Pentafluor-λ6-sulfanyl)benzyl)bicyclo[2.2.1]heptan-2-carboxamid,
(1S,2S,4R)-N-(3-(Pentafluor-λ6-sulfanyl)benzyl)bicyclo[2.2.1]heptan-2-carboxamid,
(1R,2R,4S)-rel-N-(3-(Trifluormethyl)benzyl)bicyclo[2.2.1]heptan-2-carboxamid,
(1R,2R,4S)-rel-N-(3-(Trifluormethoxy)benzyl)bicyclo[2.2.1]heptan-2-carboxamid,
(1S,2S,4R)-N-(3-(Trifluormethyl)benzyl)bicyclo[2.2.1]heptan-2-carboxamid,
(1S,2S,4R)-N-(3-(Trifluormethoxy)benzyl)bicyclo[2.2.1]-heptan-2-carboxamid,
p-Chlor-N-(4-trifluormethoxy)benzyl)benzamid,
p-Chlor-N-(4-(pentafluorsulfanyl)benzyl)benzamid,
p-Fluor-N-(4-trifluormethoxy)benzyl)benzamid,
p-Fluor-N-(4-(pentafluorsulfanyl)benzyl)benzamid,
p-Chlor-N-(4-(trifluormethyl)benzyl)benzamid und
p-Fluor-N-(4-(trifluormethyl)benzyl)benzamid.

6. Nichtwässrige Gelzusammensetzung nach Anspruch 5, wobei:
- Rizinusöl in einer Menge von 85 bis 95 Gew.-%, bezogen auf das Gesamtgewicht der Zusammensetzung, vorliegt,
- Labrafil^{®} in einer Menge von 2 bis 6 Gew.-%, bezogen auf das Gesamtgewicht der Zusammensetzung, vorliegt,
- mindestens ein Verdickungsmittel, vorzugsweise Siliciumdioxid, weiter bevorzugt kolloidales Siliciumdioxid, in einer Menge von 1 bis 6 Gew.-%, bezogen auf das Gesamtgewicht der Zusammensetzung, vorliegt,
- die Viskosität der Zusammensetzung bei einer Temperatur von 25 °C zwischen 1400 und 2400 mPas, vorzugsweise zwischen 1400 und 1800 mPas, liegt,
- die nichtwässrige Gelzusammensetzung ferner mindestens einen pharmazeutischen Wirkstoff mit einer mittleren Teilchengröße von 0,01 bis 100 µm umfasst und
- der mindestens eine pharmazeutische Wirkstoff ausgewählt ist aus einer Gruppe umfassend
(1R,2R,4S)-rel-N-(3-(Pentafluor-λ6-sulfanyl)benzyl)bicyclo[2.2.1]heptan-2-carboxamid,
(1S,2S,4R)-N-(3-(Pentafluor-λ6-sulfanyl)benzyl)bicyclo[2.2.1]heptan-2-carboxamid,
(1R,2R,4S)-rel-N-(3-(Trifluormethyl)benzyl)bicyclo[2.2.1]heptan-2-carboxamid,
(1R,2R,4S)-rel-N-(3-(Trifluormethoxy)benzyl)bicyclo[2.2.1]heptan-2-carboxamid,
(1S,2S,4R)-N-(3-(Trifluormethyl)benzyl)bicyclo[2.2.1]heptan-2-carboxamid,
(1S,2S,4R)-N-(3-(Trifluormethoxy)benzyl)bicyclo[2.2.1]-heptan-2-carboxamid,
p-Chlor-N-(4-trifluormethoxy)benzyl)benzamid,
p-Chlor-N-(4-(pentafluorsulfanyl)benzyl)benzamid,
p-Fluor-N-(4-trifluormethoxy)benzyl)benzamid,
p-Fluor-N-(4-(pentafluorsulfanyl)benzyl)benzamid,
p-Chlor-N-(4-(trifluormethyl)benzyl)benzamid und
p-Fluor-N-(4-(trifluormethyl)benzyl)benzamid.

7. Nichtwässrige Gelzusammensetzung nach Anspruch 5, bestehend aus:
- Rizinusöl, das in einer Menge von 85 bis 95 Gew.-%, bezogen auf das Gesamtgewicht der Zusammensetzung, vorliegt,
- Labrafil^{®}, das in einer Menge von 2 bis 6 Gew.-%, bezogen auf das Gesamtgewicht der Zusammensetzung, vorliegt,
- mindestens einem Verdickungsmittel, vorzugsweise Siliciumdioxid, weiter bevorzugt kolloidalem Siliciumdioxid, das in einer Menge von 1 bis 6 Gew.-%, bezogen auf das Gesamtgewicht der Zusammensetzung, vorliegt,
- mindestens einem pharmazeutischen Wirkstoff mit einer mittleren Teilchengröße von 0,01 bis 100 µm, und
- wobei der mindestens eine pharmazeutische Wirkstoff ausgewählt ist aus einer Gruppe umfassend
(1R,2R,4S)-rel-N-(3-(Pentafluor-λ6-sulfanyl)benzyl)bicyclo[2.2.1]heptan-2-carboxamid,
(1S,2S,4R)-N-(3-(Pentafluor-λ6-sulfanyl)benzyl)bicyclo[2.2.1]heptan-2-carboxamid,
(1R,2R,4S)-rel-N-(3-(Trifluormethyl)benzyl)bicyclo[2.2.1]heptan-2-carboxamid,
(1R,2R,4S)-rel-N-(3-(Trifluormethoxy)benzyl)bicyclo[2.2.1]heptan-2-carboxamid,
(1S,2S,4R)-N-(3-(Trifluormethyl)benzyl)bicyclo[2.2.1]heptan-2-carboxamid,
(1S,2S,4R)-N-(3-(Trifluormethoxy)benzyl)bicyclo[2.2.1]-heptan-2-carboxamid,
p-Chlor-N-(4-trifluormethoxy)benzyl)benzamid,
p-Chlor-N-(4-(pentafluorsulfanyl)benzyl)benzamid,
p-Fluor-N-(4-trifluormethoxy)benzyl)benzamid,
p-Fluor-N-(4-(pentafluorsulfanyl)benzyl)benzamid,
p-Chlor-N-(4-(trifluormethyl)benzyl)benzamid und
p-Fluor-N-(4-(trifluormethyl)benzyl)benzamid, wobei
- die Viskosität der Zusammensetzung bei einer Temperatur von 25 °C zwischen 1400 und 2400 mPas, vorzugsweise zwischen 1400 und 1800 mPas, liegt.

8. Spritze, wobei die Spritze mit einer nichtwässrigen Gelzusammensetzung nach einem der Ansprüche 1 bis 7 gefüllt ist.

9. Nichtwässrige Gelzusammensetzung nach einem der Ansprüche 1 bis 5 zur Verwendung als pharmazeutische Zusammensetzung oder Medikament, wobei die Verwendung zur Prävention oder Behandlung von Innenohrerkrankungen dient, wobei vorzugsweise die nichtwässrige Gelzusammensetzung zur transtympanischen Verabreichung bereitgestellt wird.

## Revendications

1. Composition de gel non aqueuse pour administration transtympanique comprenant
- huile de ricin, présente en une quantité de 85 à 95 % en poids par rapport au poids total de la composition,
- Labrafil^{®}, présent en une quantité de 2 à 6 % en poids par rapport au poids total de la composition,
- au moins un épaississant, de préférence du dioxyde de silicium, plus préférablement du dioxyde de silicium colloïdal, présent en une quantité de 1 à 6 % en poids par rapport au poids total de la composition, et dans laquelle
la viscosité de la composition à une température de 25 °C est comprise entre 1 400 et 2 400 mPas, de préférence entre 1 400 et 1 800 mPas,
la composition de gel non aqueuse comprend en outre au moins un agent pharmaceutiquement actif, ayant une taille moyenne de particule de 0,01 à 100 µm,
dans laquelle l'au moins un agent pharmaceutiquement actif a la formule I : 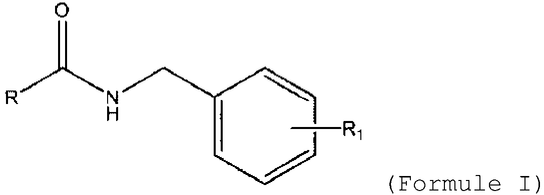 dans laquelle
- R est un groupe cycloalkyle non substitué, en particulier un groupe bicycloalkyle,
un groupe phényle non substitué ou substitué, ou un groupe thiényle non substitué ou substitué, ledit groupe thiényle ou groupe phényle substitué étant substitué par au moins un halogène, de préférence au moins un atome de F ou au moins un atome de Cl, et
- R₁ est F, SF₅, CF₃ ou OCF₃.

2. Composition de gel non aqueuse selon la revendication 1, dans laquelle ledit au moins un agent pharmaceutiquement actif est présent en une quantité de 0,05 à 10 % en poids, préférablement de 0,1 à 10 % en poids, plus préférablement de 0,5 à 5 % en poids par rapport au poids total de la composition,

3. Composition de gel non aqueuse selon la revendication 1 ou 2, dans laquelle ledit au moins un agent pharmaceutiquement actif a une taille moyenne de particule de 5 à 80 µm, de préférence de 20 à 50 µm.

4. Composition de gel non aqueuse selon l'une quelconque des revendications précédentes, dans laquelle l'au moins un agent pharmaceutiquement actif a une solubilité (faible) dans l'eau à température ambiante < 1 mg/ml, de préférence < 0,1 mg/ml.

5. Composition de gel non aqueuse selon l'une quelconque des revendications précédentes, dans laquelle l'au moins un agent pharmaceutiquement actif est choisi dans un groupe comprenant
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluorméthyl)benzyl)bicyclo[2.2.1]heptane-2-carboxamide,
(1R,2R,4S)-rel-N-(3-(trifluorométhoxy)benzyl)bicyclo[2.2.1]heptane-2-carboxamide,
(1S,2S,4R)-N-(3-(trifluorométhyl)benzyl)bicyclo[2.2.1]heptane-2-carboxamide,
(1S,2S,4R)-N-(3-(trifluorométhoxy)benzyl)bicyclo[2.2.1]heptane-2-carboxamide,
p-Chloro-N-(4-trifluorométhoxy)benzyl)benzamide,
p-Chloro-N-(4-(pentafluorosulfanyl)benzyl)benzamide,
p-Fluoro-N-(4-trifluorométhoxy)benzyl)benzamide,
p-Fluoro-N-(4-(pentafluorosulfanyl)benzyl)benzamide,
p-Chloro-N-(4-(trifluorométhyl)benzyl)benzamide, et
p-Fluoro-N-(4-(trifluorométhyl)benzyl)benzamide.

6. Composition de gel non aqueuse selon la revendication 5, dans laquelle :
- l'huile de ricin est présente en une quantité de 85 à 95 % en poids par rapport au poids total de la composition,
- Labrafil^{®}, est présent en une quantité de 2 à 6 % en poids par rapport au poids total de la composition,
- au moins un épaississant, de préférence du dioxyde de silicium, plus préférablement du dioxyde de silicium colloïdal, présent en une quantité de 1 à 6 % en poids par rapport au poids total de la composition,
- la viscosité de la composition à une température de 25 °C est comprise entre 1 400 et 2 400 mPas, de préférence entre 1 400 et 1 800 mPas,
- la composition de gel non aqueuse comprend en outre au moins un agent pharmaceutiquement actif, ayant une taille moyenne de particule de 0,01 à 100 µm et
- l'au moins un agent pharmaceutiquement actif est choisi dans un groupe comprenant
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluorméthyl)benzyl)bicyclo[2.2.1]heptane-2-carboxamide,
(1R,2R,4S)-rel-N-(3-(trifluorométhoxy)benzyl)bicyclo[2.2.1]heptane-2-carboxamide,
(1S,2S,4R)-N-(3-(trifluorométhyl)benzyl)bicyclo[2.2.1]heptane-2-carboxamide,
(1S,2S,4R)-N-(3-(trifluorométhoxy)benzyl)bicyclo[2.2.1]heptane-2-carboxamide,
p-Chloro-N-(4-trifluorométhoxy)benzyl)benzamide,
p-Chloro-N-(4-(pentafluorosulfanyl)benzyl)benzamide,
p-Fluoro-N-(4-trifluorométhoxy)benzyl)benzamide,
p-Fluoro-N-(4-(pentafluorosulfanyl)benzyl)benzamide,
p-Chloro-N-(4-(trifluorométhyl)benzyl)benzamide, et
p-Fluoro-N-(4-(trifluorométhyl)benzyl)benzamide.

7. Composition de gel non aqueuse selon la revendication 5, constituée par :
- une huile de ricin présente en une quantité de 85 à 95 % en poids par rapport au poids total de la composition,
- Labrafil^{®}, présent en une quantité de 2 à 6 % en poids par rapport au poids total de la composition,
- au moins un épaississant, de préférence du dioxyde de silicium, plus préférablement du dioxyde de silicium colloïdal, présent en une quantité de 1 à 6 % en poids par rapport au poids total de la composition,
- au moins un agent pharmaceutiquement actif, présentant une taille moyenne de particule de 0,01 à 100 µm et
- l'au moins un agent pharmaceutiquement actif est choisi dans un groupe comprenant
(1R,2R,4S)-rel-N-(3-(pentafluoro-λ6-sulfanyl)benzyl)bicyclo[2.2.1]heptane-2-carboxamide,
(1S,2S,4R)-N-(3-(pentafluoro-λ6-sulfanyl)benzyl)bicyclo[2.2.1]heptane-2-carboxamide,
(1R,2R,4S)-rel-N-(3-(trifluorméthyl)benzyl)bicyclo[2.2.1]heptane-2-carboxamide,
(1R,2R,4S)-rel-N-(3-(trifluorométhoxy)benzyl)bicyclo[2.2.1]heptane-2-carboxamide,
(1S,2S,4R)-N-(3-(trifluorométhyl)benzyl)bicyclo[2.2.1]heptane-2-carboxamide,
(1S,2S,4R)-N-(3-(trifluorométhoxy)benzyl)bicyclo[2.2.1]heptane-2-carboxamide,
p-Chloro-N-(4-trifluorométhoxy)benzyl)benzamide,
p-Chloro-N-(4-(pentafluorosulfanyl)benzyl)benzamide,
p-Fluoro-N-(4-trifluorométhoxy)benzyl)benzamide,
p-Fluoro-N-(4-(pentafluorosulfanyl)benzyl)benzamide,
p-Chloro-N-(4-(trifluorométhyl)benzyl)benzamide, et
p-Fluoro-N-(4-(trifluorométhyl)benzyl)benzamide, dans
laquelle
- la viscosité de la composition à une température de 25 °C est comprise entre 1 400 et 2 400 mPas, de préférence entre 1 400 et 1 800 mPas.

8. Seringue, dans laquelle la seringue est remplie avec une composition de gel non aqueuse selon l'une quelconque des revendications 1 à 7.

9. Composition de gel non aqueuse selon l'une quelconque des revendications 1 à 5, destinée à être utilisée en tant que composition pharmaceutique ou médicament, dans laquelle ladite utilisation est destinée à la prévention ou au traitement de maladies de l'oreille interne, dans laquelle ladite composition de gel non aqueuse est de préférence fournie pour une administration transtympanique.
